(19) Europäisches Patentamt
European Patent Office
Office européen des brevets

(11) Veröffentlichungsnummer : **0 056 615**
**B1**

(12) # EUROPÄISCHE PATENTSCHRIFT

(45) Veröffentlichungstag der Patentschrift : **13.06.84**

(51) Int. Cl.³ : **C 07 C103/183**

(21) Anmeldenummer : **82100196.3**

(22) Anmeldetag : **13.01.82**

(54) **Verfahren zur Herstellung von Carnitinamid.**

(30) Priorität : **21.01.81 CH 363/81**

(43) Veröffentlichungstag der Anmeldung : **28.07.82 Patentblatt 82/30**

(45) Bekanntmachung des Hinweises auf die Patenterteilung : **13.06.84 Patentblatt 84/24**

(84) Benannte Vertragsstaaten : **AT BE CH DE FR GB IT LI LU NL SE**

(56) Entgegenhaltungen :
**DE-A- 2 542 227**
**CHEMICAL ABSTRACTS, Band 59, Nr. 10, 11. November 1963, Spalte 11660g, Columbus, Ohio, USA**

(73) Patentinhaber : **LONZA AG**
**Gampel/Wallis (CH)**

(72) Erfinder : **Tenud, Leander, Dr.**
**Balfrinstrasse 23**
**CH-Visp (Kanton Wallis) (CH)**
Erfinder : **Blum, René, Dr.**
**Bollwerkstrasse 60**
**CH-Binningen (Kanton Baselland) (CH)**

(74) Vertreter : **Weinhold, Peter, Dr.**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8**
**D-8000 München 40 (DE)**

**EP 0 056 615 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von Carnitinamid in Form des Chlorids.

Es ist bekannt, Carnitinamidchlorid aus Carnitinnitril herzustellen.

Bei einer Methode wird laut der belgischen Patentschrift 659 194 das aus Epichlorhydrin hergestellte Carnitinnitril mittels Hydrolyse mit konz. Salzsäure bei 15 bis 55 °C während 46 bis 48 Stunden umgesetzt. Dabei entstehen als Nebenprodukte Carnitinhydrochlorid und Ammonchlorid, die die Qualität des gewünschten Produktes negativ beeinflussen.

Ein anderes Procedere, welches in der japanischen Patentanmeldung Nr. 23 (1963) beschrieben wird, besteht aus der Behandlung von Carnitinnitrilchlorid mit einem Ueberschuss an Wasserstoffsuperoxid in basischem Milieu. Diesem Prozess haftet, trotz guter Ausbeute, der grosse Nachteil der Unwirtschaftlichkeit an. Wasserstoffsuperoxid ist relativ teuer und es muss mit einem grossen Ueberschuss gearbeitet werden.

In der japanischen Patentanmeldung Nr. 24 (1963) wird ein Verfahren zur Herstellung von Amiden des Carnitins beschrieben, bei dem beispielsweise Carnitinäthylesterchlorid mit Ammoniak umgesetzt wird.

Bei den Verfahren der japanischen Patentanmeldung wird also von Carnitinverbindungen ausgegangen. Des weiteren muß bei dem letztgenannten Verfahren zunächst das Carnitinäthylesterchlorid z. B. aus Carnitinhydrochlorid durch Umsetzung mit äthanolischer Salzsäure hergestellt werden.

Ferner wird in der japanischen Patentanmeldung Nr. 24 (1963) zur Durchführung der Umsetzung des Carnitinäthylesterchlorids mit Ammoniak eine Methode gewählt, die — als Stufe des erfindungsgemäßen Verfahrens angewendet — zu erheblichen Mengen (etwa 5-7 %) an unerwünschtem Carnitinhydrochlorid im erhaltenen Produkt führen würde.

Aufgabe der vorliegenden Erfindung ist die Schaffung eines Verfahrens, mit dem es möglich ist, — ausgehend vom 4-Chloracetessigsäureäthylester — ohne jegliche Isolierung von Zwischenprodukten, praktisch in einem Arbeitsgang, das Carnitinamid in Form eines Chlorids in guter Ausbeute und in qualitativ hochwertiger Form zu erhalten. Gegenstand der Erfindung ist ein Verfahren zur Herstellung von Carnitinamid in Form des Chlorids, das dadurch gekennzeichnet ist, daß man 4-Chloracetessigsäureäthylester mit Trimethylamin zum entsprechenden Dehydrocarnitinester umsetzt, diesen anschließend mit Hilfe eines Edelmetall-Katalysators, vorzugsweise eines Pt/C-Katalysators, zum Carnitinethylester unter Druck hydriert und diesen ohne ihn zu isolieren in einem Autoklaven bei − 30° bis + 10 °C mit Ammoniak versetzt, alles auf 40 bis 80 °C erwärmt, das Gemisch bei der genannten Temperatur bei einem Ammoniak-Druck von 8,8-24,5 bar (8 bis 24 atü) rührt und das so erhaltene Produkt nach dem Abkühlen auf Raumtemperatur und Aufheben des Überdruckes filtriert, vorzugsweise bei 30 bis 50 °C und bei Druckverhältnissen von 0,02 bis 0,033 bar (15 bis 25 Torr) trocknet.

Die Vorteile, die diese Erfindung gegenüber dem Stand der Technik bringt, sind die folgenden :

— Direktes Verfahren ohne Isolation von Zwischenprodukten.

— Der Verfahrensablauf erlaubt eine Entfernung des anfallenden, im Endprodukt störenden Ammonchlorids.

— Das Endprodukt ist frei von Carnitinhydrochlorid.

— Die Raum/Zeit-Ausbeute ist gross.

Erfindungsgemäss wird das gesteckte Ziel erreicht, indem man also nach folgendem Schema vorgeht

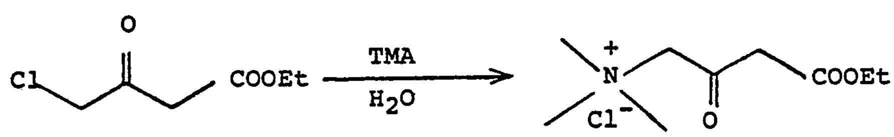


(I) (II)

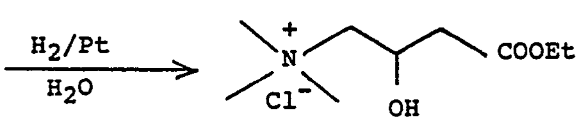


(III)

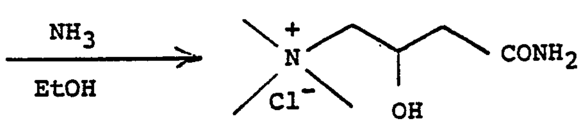


(IV)

I = 4-Chloracetessigsäureäthylester
II = Dehydrocarnitinester
III = Carnitinester
IV = Carnitinamidchlorid
TMA = Trimethylamin

und welches ein Verfahren darstellt, das dadurch gekennzeichnet ist, dass der als Zwischenprodukt anfallende Carnitin-ester nicht isoliert werden muss.

Zweckmässig wird dieses geradlinige und direkte Verfahren wie folgt durchgeführt :

Die vorgesehene Menge Trimethylamin (TMA) vorzugsweise in wässriger Lösung wird vorgelegt und bei ca. 10 °C der 4-Chloracetessigsäureäthylester langsam zudosiert. Aus dem gelblichen Reaktionsgemisch, welches mittels Einengung auf einen pH von 7 bis 8 gebracht worden ist, wird das überschüssige TMA auskondensiert und zurückgewonnen. Mit konz. Salzsäure wird das Ganze leicht angesäuert, filtriert und in einem Autoklaven unter Druck katalytisch hydriert. Als Katalysator wird Platin auf aktivkohle bevorzugt. Der vom Katalysator befreite, eingeengte und bei 30-40 °C 0,13-0,27 mbar (0,1-0,2 Torr) getrocknete Rückstand wird in Aethylalkohol gelöst, während ca. 1 Stunde bei Raumtemperatur mit gasförmigem Ammoniak gesättigt und das anfallende Ammoniumchlorid herausfiltriert. Dem Filtrat wird anschliessend in einem Autoklaven bei − 30 °C Ammoniak zugegeben und das Ganze bei 40 bis 80 °C, vorzugsweise bei 50 bis 70 °C, bei einem Ammoniak-Druck von 8,8 bis 24,5 bar (8 bis 24 atü) vorzugsweise bei 9,8 bis 15,7 bar (9 bis 15 atü), 3 bis 20 Stunden, vorzugsweise 4 bis 6 Stunden gerührt. Nach Ende der Reaktion wird nach Abkühlen des Reaktionsgemisches auf Raumtemperatur das ausgefallene Carnitinamidchlorid herausfiltriert, mit Aethylalkohol gewaschen und im Trockenschrank im allgemeinen bei 30 bis 50 °C, zweckmässig bei 35 bis 45 °C, und im allgemeinen bei 0,02 bis 0,033 bar (15 bis 25 Torr), vorzugsweise bei 0,013 bis 0,027 bar (10 bis 20 Torr), bis zur Gewichtskonstanz getrocknet. Die Ausbeuten, die so erzielt werden, belaufen sich auf ca. 80 % 100 %iges Canitinamid, berechnet auf den Eingesetzten 100 %igen 4-Chloracetessigsäureäthylester.

## Beispiel 1

In einem 2,5-Liter-Doppelmantelgefäss, ausgerüstet mit Ablasshahn, mechanischem Rührer und Thermometer, wurden 1 062,7 g Trimethylamin (44,5 %ig in Wasser = 472,9 g 100 %ig = 8 Mol) vorgelegt und auf 10 °C gekühlt. Mittels einer Dosierpumpe und in die TMA-Lösung eintauchendem Schlauch wurden dann 272,8 g 4-Chloracetessigsäureäthylester (96,0 %ig = 261,9 g 100 %ig = 1,59 Mol) während 3 Stunden bei 10 bis 12 °C zudosiert und das gelbliche Reaktionsgemisch bei 40 °C und 0,033 bar (25 Torr) so eingeengt, bis der pH-Wert 7,6 betrug. Das überschüssige TMA wurde anschliessend mittels drei Kühlfallen auskondensiert und zurückgewonnen. Nach einem leichten Absenken des pH-Wertes mit 65 ml 36 %iger Salzsäure auf 6 und dem Herausfiltrieren des anfallenden gelben Niederschlages (2,5-Diäthoxycarbonyl-1,4-cyclohexandien) wurde die Reaktionslösung in einem mit Rührwerk ausgestatteten 2-Liter-Autoklaven während 7 Stunden bei 10 °C und 11,8 bar (11 atü) Druck mit Hilfe von 8,8 g Platin/Aktivkohle-Katalysator hydriert.

Nachdem der Katalysator nach beendeter Hydrierung abfiltriert worden ist, wurde das Filtrat in einem Rotavapor bei 50 °C und 30 Torr vollständig eingeengt, anschliessend im Hochvakuum [5 Stunden bei 30 bis 40 °C, 0,13 bis 0,27 mbar (0,1 bis 0,2 Torr)] getrocknet und der Rückstand in 295 g absolutem Aethylalkohol gelöst. Ein Teil dieser Lösung, in diesem Fall eine 154,9 g Rohcarnitinester beinhaltende Menge (entsprechend 0,4 Mol 4-Chloracetessigsäure-äthylester) wurden während 1 Stunde bei Raumtemperatur mit gasförmigem Ammoniak gesättigt. 7,7 g Ammoniumchlorid fielen aus und wurden abfiltriert. Das Filtrat wurde anschliessend in einem mit Rührwerk ausgerüsteten 1-Liter-Autoklaven eingefüllt, auf − 30 °C abgekühlt und mit 73 g Ammoniak versetzt. Nach dem Verschliessen des Druckreaktors wurde die Temperatur auf 60 °C angehoben und das Reaktionsgemisch mit 250 Umdrehungen pro Minute 5 Stunden lang gerührt. Beim Erreichen der genannten 60 °C wurde soviel Ammoniak abgelassen, dass der Arbeitsdruck 10,8 bar (10 atü) betrug.

Am Ende der Reaktion wurde das ganze System auf Raumtemperatur abgekühlt und der Autoklav entspannt.

Das ausgefallene Carnitinamidchlorid wurde abfiltriert, zweimal mit 100 g Aethylalkohol bei 20 °C gewaschen und in einem Trockenschrank bei 40 °C und 0,027 bar (20 Torr) bis zur Gewichtskonstanz getrocknet.

Die Ausbeute betrug 63,0 g 99,1 %iges Carnitinamidchlorid, was 62,4 g 100 %iger Substanz entsprach und dies ist 79,3 % der Theorie, bezogen auf 100 %igen 4-Chloracetessigsäureäthylester, gleichzusetzen.

## Beispiele 2-8

Die Beispiele wurden nach demselben Prinzip wie das Beispiel 1 durchgeführt. Die Abweichungen sind aus der nachstehenden Tabelle ersichtlich.

Tabelle

| Beispiel Nr. | Rohester $H_2O$-Gehalt Gew.% | Esterkonz. Gew.% | Druck bar (atü) | Ammoniak Moläq. | T °C | Zeit h | *Ausbeute % |
|---|---|---|---|---|---|---|---|
| 2 | 3,1 | 44 | 8,8 (8) | 11 | 45 | 20 | 82,5 |
| 3 | 2,0 | 47 | 10,8(10) | 10 | 60 | 20 | 85,3 |
| 4 | 1,7 | 35 | 24,5(24) | 30 | 80 | 3 | 79,3 |
| 5 | 2,5 | 35 | 10,8(10) | 20 | 40 | 7 | 79,7 |
| 6 | 3,2 | 45 | 10,8(10) | 7 | 80 | 3 | 78,4 |
| 7 | 6,3 | 40 | 10,8(10) | 10 | 60 | 5 | 79,6 |
| 8 | 2,9 | 41 | 10,8(10) | 22 | 40 | 14 | 82,2 |

(*) Ausbeute bezogen auf 100 %igen 4-Chloracetessigsäureäthylester.
Gehalt : 99 %

**Ansprüche**

1. Verfahren zur Herstellung von Carnitinamid in Form des Chlorids, dadurch gekennzeichnet, dadurch gekennzeichnet, daß man 4-Chlor-acetessigsäureethylester mit Trimethylamin zum entsprechenden Dehydrocarnitinester umsetzt, diesen anschließend mit Hilfe eines Edelmetall-Katalysators, vorzugsweise eines Pt/C-Katalysators, zum Carnitinethylester unter Druck hydriert und diesen ohne ihn zu isolieren in einem Autoklaven bei − 30° bis + 10°C mit Ammoniak versetzt, alles auf 40 bis 80 °C erwärmt, das Gemisch bei der genannten Temperatur bei einem Ammoniak-Druck von 8,8 bis 24,5 bar (8 bis 24 Atü) rührt und das so erhaltene Produkt nach dem Abkühlen auf Raumtemperatur und Aufheben des Überdruckes filtriert, vorzugsweise bei 30 bis 50 °C und bei Druckverhältnissen von 0,02 bis 0,033 bar (15 bis 25 Torr) trocknet.

2. Verfahren nach Anspruch 1, dadurch gekennzeichnet, daß man die Ammonolyse bei einer Temperatur von 50 bis 70 °C durchführt.

3. Verfahren nach Ansprüche 1 und 2, dadurch gekennzeichnet, daß die Rührzeit im Autoklaven 4 bis 6 Stunden umfaßt.

4. Verfahren nach Ansprüche 1 bis 3, dadurch gekennzeichnet, daß der Ammoniak-Überdruck im Autoklaven 9,8 bis 15,7 bar (9 bis 15 atü) ausmacht.

5. Verfahren nach Ansprüche 1 bis 4, dadurch gekennzeichnet, daß der abschließende Trocknungsprozess bei 35 bis 45 °C und in einem Vakuum von 0,013 bis 0,027 bar (10 bis 20 Torr) durchgeführt wird.

**Claims**

1. Process for the preparation of carnitine amide in the form of the chloride, characterized in reacting ethyl 4-chloro aceto acetate with trimethylamine to the corresponding dehydrocarnitionate, subsequently hydrogenating said ester under pressure to ethyl carnitinoate by means of a noble metal catalyst, preferably of a Pt/C-catalyst, adding to this ester, without isolating it, ammonia in an autoclave at − 30 to + 10 °C, heating the whole mixture to 40 to 80 °C, stirring the mixture at said temperature under an ammonia pressure of from 8.8 to 24.5 bar (8 to 24 atü), filtering the thus obtained product after cooling and lifting the gauge pressure, and drying it at 30 to 50 °C and under pressure conditions from 0.02 to 0.033 bar (15 to 25 torr).

2. Process according to claim 1, characterized in that the ammonolysis is carried out at a temperature of from 50 to 70 °C.

3. Process according to claims 1 and 2, characterized in that the stirring time in the autoclave comprises 4 to 6 hours.

4. Process according to claims 1 to 3, characterized in that the ammonia gauge pressure in the

autoclave is 9.8 to 15.7 bar (9 to 15 atü).

5. Process according to claims 1 to 4, characterized in that the final drying procedure is carried out at 35 to 45 °C and under a vacuum of 0.013 to 0.027 bar (10 to 30 torr).

**Revendications**

1. Procédé de préparation du carnitinamide sous forme de chlorure, caractérisé en ce que l'on fait réagir le 4-chloracétate d'éthyle avec la triméthylamine, pour donner l'ester l'ester de déshydrocarnitine correspondant, on hydrogène ensuite celui-ci sous pression à l'aide d'un catalyseur à base de métal noble, de préférence un catalyseur au Pt/C, pour donner l'ester éthylique de la carnitine auquel on ajoute, sans l'isoler, dans un autoclave à une température de − 30 à + 10 °C, de l'ammoniac, on chauffe le tout à une température de 40 à 80 °C, on agite le mélange à la température indiquée sous une pression d'ammoniac de 8,8 à 24,5 bars (8 à 24 atmosphères effectives), on filtre le produit obtenu après refroidissement à température ambiante et élimination de la surpression et on le sèche de préférence à une température de 30 à 50 °C sous un taux de compression de 0,02 à 0,033 bar (15 à 25 torrs).

2. Procédé selon la revendication 1, caractérisé en ce que l'on effectue l'ammoniolyse à une température de 50 à 70°C.

3. Procédé selon les revendications 1 et 2, caractérisé en ce que la durée d'agitation à l'autoclave est de 4 à 6 heures.

4. Procédé selon les revendications 1 à 3, caractérisé en ce que la surpression d'ammoniac dans l'autoclave est de 9,8 à 15,7 bars (9 à 15 atmosphères effectives).

5. Procédé selon les revendications 1 à 4, caractérisé en ce que l'opération finale de séchage est effectuée à une température de 35 à 45 °C et sous un vide de 0,013 à 0,027 bar (10 à 20 torrs).